# EUROPEAN PATENT SPECIFICATION

(11) **EP 3 666 262 B1**
(45) Date of publication and mention of the grant of the patent: **10.11.2021**
(21) Application number: 18211446.2
(22) Date of filing: 10.12.2018
(51) Int. Cl.: A61K 9/28, A61K 45/06, A61K 31/19, A61K 31/201, A61K 31/353, A61K 31/575, A61K 31/7016, A61K 31/704

(54) **PHARMACEUTICAL ORAL DOSAGE FORMS FOR TREATMENT OF METABOLIC DISORDERS AND RELATED DISEASES THROUGH ORCHESTRATED RELEASE OF ENTEROKINES**
PHARMAZEUTISCHE ORALE DARREICHUNGSFORMEN ZUR BEHANDLUNG VON STOFFWECHSELSTÖRUNGEN UND VERWANDTEN ERKRANKUNGEN DURCH ORCHESTRIERTE FREISETZUNG VON ENTEROKINEN
FORMES POSOLOGIQUES ORALES PHARMACEUTIQUES POUR LE TRAITEMENT DES TROUBLES MÉTABOLIQUES ET DES MALADIES CONNEXES PAR LA LIBÉRATION ORCHESTRÉE D'ENTÉROKINASES

(43) Date of publication of application: 17.06.2020
(73) Proprietor: Aphaia IP AG, 6300 Zug (CH)
(72) Inventor: Deusch, Kai, 82057 Icking (DE); Bolz, Steffen-Sebastian, 82211 Herrsching (DE)
(74) Representative: Habermann, Hruschka & Schnabel

(56) References cited:
- EP-A1- 2 659 881
- US-A1- 2014 294 951

## Description

The present invention relates to pharmaceutical oral dosage forms releasing compounds in a specific part of the small intestine of a subject, wherein said compounds stimulate enteroendocrine cells in the subject's jejunum and lower small intestine to release one or more enterokines. The present invention also relates to a method of producing such pharmaceutical oral dosage forms. The pharmaceutical oral dosage forms of the invention are particularly for use in the treatment and prevention of metabolic conditions or diseases, osteoporosis, malabsorption conditions, neurodegenerative diseases, conditions of impaired gastro-intestinal function and cardiovascular diseases.

It has early been recognised that delivery of a nutritional substance to the ileum through use of an enteric dosage form of the nutritional substance leads to satiety of a mammalian subject (US 5753253 A). More recently, specific delivery of a nutritional substance, in particular glucose, to the ileum of a subject, in particular dosage forms of a nutritional substance like glucose, which dosage forms release at least 50 % of the administered substance in the ileum of the subject, was suggested for treatment of metabolic diseases like type 2 diabetes mellitus, metabolic syndrome, insulin resistance, obesity, non-alcoholic fatty liver disease (NAFLD), non-alcoholic steatohepatosis (NASH) and other conditions (WO 2010/027498 A2, WO 2012/118712 A2, US 2014/294951 A1).

The technical problem underlying the present invention is to provide improved preventive and therapeutic measures against metabolic and cardiovascular disorders as well as other conditions relating thereto.

The solution to the above technical problem is provided by the embodiments of the present invention as disclosed in the claims, the present description and the accompanying figures.

In particular, the present invention provides pharmaceutical oral dosage forms comprising a core and a pH-sensitive enteric coating, wherein the core comprises at least one nutrient compound stimulating enteroendocrine cells to release at least one enterokine, and at least one disintegrant providing a burst release of the ingredients of the core when the coating is substantially degraded and/or dissolved, and wherein the coating comprises a pH sensitive polymer which substantially degrades and/or dissolves at a pH value of about 5.5 to about 7.5, wherein the dosage forms have a size of less than 3 mm, based on the largest dimension of the oral dosage forms, and release said nutrient compound in the terminal jejunum of a subject.

A "pharmaceutical oral dosage from" according to the invention is a pharmaceutical composition typically formulated such that it is suitable for oral administration to a subject, preferably a human patient. Preferred oral dosage forms of the invention include tablets, capsules, pellets and granules.

An "enteroendocrine cell" (EEC) as used in the present invention, is a cell present in a subject's, preferably, human subject's, intestine, in particular in the small intestine's mucosa, and secretes one or more enterokines upon receiving an appropriate trigger by a nutritional component. There are several types of enteroendocrine cells which can be addressed by the active compound or compounds released by the pharmaceutical dosage form of the invention (for a review of enteroendorine cells of the lower gastrointestinal tract, see, for example, Gunawardene et al. (2011) Int. J. Exp. Pathol. 92, 219-231, and Latorre et al. (2017) Neurogastrol. Motil 28 (5), 620-630). In particular, preferred enteroendocrine cells in the context of the invention are I cells, K cells and L cells, with L cells being most preferred.

According to the invention, an "enterokine" is a hormone secreted by EECs in the gastro-intestinal system. Preferred enterokines are the incretins, i.e. hormones regulating the blood sugar level upon intake of nutrition, preferably GLP-1, GLP-2 GIP and PYY, more preferably GLP-1 and PYY. Other preferred enterokines secreted by the EEC(s) through release of the active compound(s) in the pharmaceutical dosage form are CCK and neurotensin.

I cells are predominantly present in the proximal small intestine, in particular in the lower duodenum and in the jejunum, and secrete cholecystokinin (CCK) upon sensing of nutrients such as amino acids and fatty acids. CCK effects the release of bile acids from the gall bladder into the small intestine, but also promotes the release of digestive secrete from the pancreas.

K cells are found in the complete small intestine and release GIP (glucose-dependent insulinotropic peptide; also known as gastric inhibitory peptide) leading to inhibition of gastric motility and enhancement of insulin production and secretion.

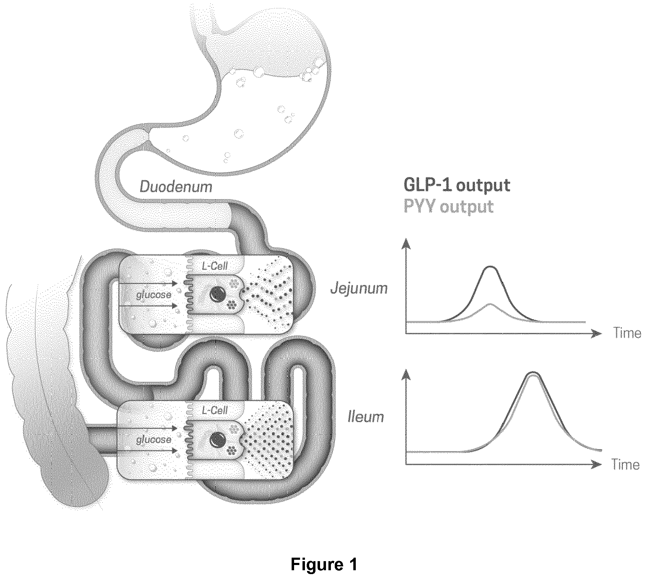

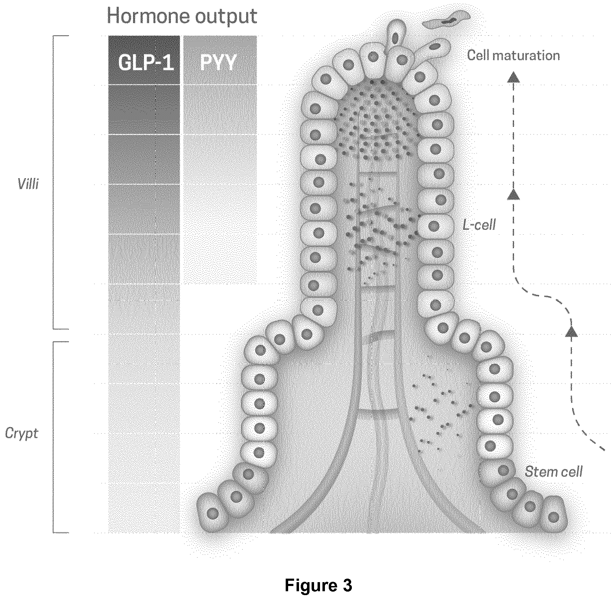

L cells are present throughout the small intestine, i.e. duodenum, jejunum, and ileum,, and release GLP-1 (glucagon-like peptide 1), GLP-2 (glucagon-like peptide 2) and PYY (peptide YY) in response to various nutrients. The concentration of L cells raises from duodenum to jejunum to ileum, and the GLP-1 release from L cells shows a gradient sloping from proximal to distal small intestine with highest GLP-1 release capacity in the terminal ileum. The gradient is not only a function of increasing number of L cells, but also a function of their maturation state and production rate of GLP-1, which also increase from proximal to distal ileum. In particular, GLP-1 is released from L cells in the crypts and on the villi of the mucosa. L cells mature from crypt to villi, and GLP-1 release capacity is highest when the L cells reach the top of the villi. L cells further secrete PYY (peptide YY) which is predominantly released in the terminal ileum. The time dependency of GLP-1 and PYY release by L cells in the jejunum and ileum in response to glucose uptake by a human subject is schematically depicted in Fig. 1. The maturation dependency of GLP-1 and PYY secretion capacity of L cells is shown schematically in Fig. 3. GLP-1 enhances nutrient-stimulated insulin secretion and inhibits glucagon secretion, gastric emptying and feeding. GLP-1 also has proliferative, neogenic and antiapoptotic effects on pancreatic β-cells. As an intestinal trophic peptide, GLP-2 stimulates cell proliferation and inhibits apoptosis in the intestinal crypt compartment, It also regulates intestinal glucose transport, food intake and gastric acid secretion and emptying. Furthermore, GLP-2 improves intestinal barrier function. PYY inhibits gastric motility and increases water and electrolyte absorption in the colon. PYY also suppresses pancreatic secretion and has been shown to reduce appetite. PYY slows gastric emptying; whereby it increases efficiency of digestion and nutrient absorption after a meal.

EECs, in particular L cells, are triggered to release enterokines such as GLP-1 and PYY through a variety of mechanisms being effected by the one or more active compound(s) in the pharmaceutical dosage form of the invention.

L cells release GLP-1 and PYY (and also GLP-2; note that GLP-1 and GLP-2 are derived from a common mRNA so that these hormones are essentially co-released; cf., for example, the review of Baggio and Drucker (2004) Best Practice & Research Clinical Endocrinology & Metabolism 18 (4), 531-554) in response to various mechanisms triggered by compounds such as nutrients. One mechanism includes intake of a carbohydrate such as glucose through glucose transporters GLUT2 and/or SGLT1. Other mechanisms rely on the binding to specialized G protein-coupled receptors such as taste receptors, fatty acid receptors, bile acid receptors, peptide receptors and amino acid receptors.

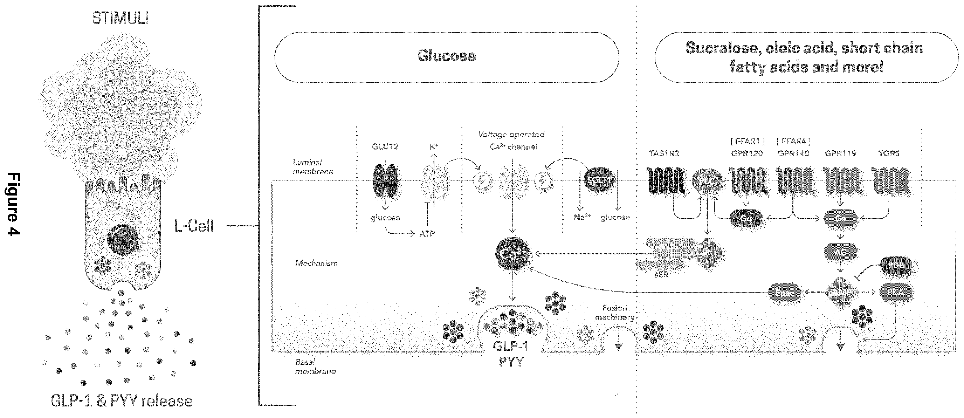

These signals, glucose transport and binding to G protein-coupled receptor, are typically transmitted in the cells by one or more of three mechanisms and lead ultimately to the release of the enterokine, in the case of L cells GLP-1 and PYY: transmembrane calcium influx, intracellular calcium release and/or intracellular cAMP increase. The various cellular mechanisms leading to release of enterokines such as GLP-1 and PYY by EECs (in that case L cells) are schematically depicted in Fig. 4.

Accordingly, the active compound(s) triggering the release of an enterokine through an EEC are selected from nutrients, and more preferred active compounds include carbohydrates, fatty acids, bile acids, peptides (including oligopeptidesl polypeptides and proteins), amino acids, alcohol amides and anthocyanins. Preferred fatty acids are fatty acids having 2 to 6 carbon atoms. Ethanolamides such as oleoylethanolamide, anandamide (N-arachidonoylethenolamide, AEA), palmitoylethanolamide, steaorylethanolamide, and derivatives of anandaminde such as prostamides, can also preferably be used as alcohol amide compounds in the inventive oral dosage forms. A particularly preferred ethanolamide is oleoylethanolamide (OEA). A preferred example of a peptidic active compound is the protein bovine serum albumin (BSA). Carbohydrates are preferably selected from glucose and sucralose, with glucose being most preferred. In a particular preferred embodiment of the invention, the pharmaceutical dosage form contains about 1 % (w/w) to about 80 % (w/w), more preferably 60 to 70 % (w/w) of active compound triggering the EECs, preferably a carbohydrate, most preferred glucose.

In a preferred embodiment, the active compound triggering the release of an enterokine through an EEC is an anthocyanin, more preferably one or more anthocyanins from *Vaccinium myrtilloides* Michx. A particularly preferred anthocyanin to be included in the core of the pharmaceutical oral dosage forms of the invention is delphinidin 3-rutinoside.

In a preferred embodiment of the invention, one or more of the above active compounds triggering enterokine release by EECs are combined in preferably synergistic combinations. Preferred combinations are combinations of a carbohydrate, preferably glucose, with sucralose and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins, such as preferably BSA) and/or one or more amino acids and/or one or more anthocyanins (preferably delphinidin 3-rutinoside).

Further preferred combinations are combinations of another carbohydrate, preferably sucralose, with glucose and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins) such as preferably BSA, and/or, one or more amino acids and/or one or more anthocyanins (such as preferably delphinidin 3-rutinoside).

Other preferred combinations are combinations of a fatty acid having 2 to 6 carbon atoms with one or more carbohydrates, preferably glucose and/or sucralose, and/or oleic acid and/or one or more bile acids and/or one or more alcohol amides, preferably one or more ethanolamides, more preferably oleoylethamolamide, and/or one or more peptides (including oligopeptides, polypeptides and proteins), preferably BSA) and/or one or more amino acids and/or one or more anthocyanins such as preferably delphinidin 3-rutinoside.

According to a further preferred embodiment, the present invention provides combinations of oleic acid with one or more fatty acids having 2 to 6 carbon atoms and/or one or more carbohydrates, preferably glucose and/or sucralose, and/or one or more bile acids and/or one or more alcohol amides, preferably one or more ethanolamides, more preferably oleoylethanolamide and/or one or more peptides (including oligopeptides, polypeptides and proteins, such as preferably BSA) and/or one or more amino acids and/or one or more anthocyanins such as preferably delphinidin 3-rutinoside.

Further preferred combinations of active compounds are combinations of one or more bile acids with one or more carbohydrates, preferably sucralose and/or glucose, and/or one or more fatty acids having 2 to 6 carbon atoms and/or oleic acid and/or one or more alcohol amides, preferably one or more ethanolamide, more preferably oleoylethanolamide, and/or one or more peptides (including oligopeptides, polypeptides and proteins, preferably BSA, and/or one or more amino acids and/or one or more anthocyanins such as preferably delphinidin 3-rutinoside.

Still further preferred combinations of active compounds are combinations of one or more alcohol amides, preferably one or more ethanolamides, more preferably oleoylethanolamide, with one or more carbohydrates, preferably sucralose and/or glucose, and/or one or more fatty acids having 2 to 6 carbon atoms and/or oleic acid and/or one or more bile acids and/or one or more peptides (including oligopeptides, polypeptides and proteins) preferably BSA, and/or one or more amino acids and/or one or more anthocyanins such as preferably delphinidin 3-rutinoside.

Other preferred combinations of active compounds are combinations of one or more peptides (including oligopeptides, polypeptides and proteins), preferably BSA, with one or more carbohydrates, preferably sucralose and/or glucose, and/or one or more fatty acids having 2 to 6 carbon atoms and/or oleic acid and/or one or more bile acids and/or one or more alcohol amide, preferably one or more ethanolamide, more preferably oleoylethanolamide, and/or one or more amino acids and/or one or more anthocyanins such as preferably delphinidin 3-rutinoside.

In other preferred embodiments of the invention, the core of the pharmaceutical oral dosage form contains a combination of one or more amino acids with one or more carbohydrates, preferably sucralose and/or glucose, and/or one or more fatty acids having 2 to 6 carbon atoms and/or oleic acid and/or one or more bile acids and/or one or more ethanolamides, more preferably oleoylethanolamide, and/or one or more peptides (including oligopeptides, polypeptides and proteins); preferably BSA, and/or one or more anthocyanins such as preferably delphinidin 3-rutinoside.

In still further preferred combinations of active compounds in the core part of the pharmaceutical oral dosage form of the invention, one or more anthocyanins, preferably delphinidin 3, is/are combined with one or more carbohydrates, preferably sucralose and/or glucose, and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins), preferably BSA, and/or one or more amino acids.

The active compound(s) in the core part of the pharmaceutical dosage form are preferably further combined with active ingredients having various functions.

Preferred examples of additional active ingredients are EEC maturation agents. As exemplified before with L cells such maturation agents typically enhance the release capacity of the EECs, such as L cells, for the relevant enterokine, in the case of L cells GLP-1 and/or PYY and/or GLP-2. Preferred EEC maturation agents, in particular L cell maturation agents, include human milk oligosaccharides (HMO) and inhibitors of NOTCH signalling such as γ-secretase inhibitors, preferably dibenzazepine. Various HMOs are commercially available (Jennewein Biotechnologie GmbH, Rheinbreitbach, Germany) and preferred HMOs in the context of the invention include, but are not limited, e.g. 2'-fucosyllactose, 3-fucosyllactose, 6'-sialyllactose and lacto-N-neotetraose as well as mixtures thereof.

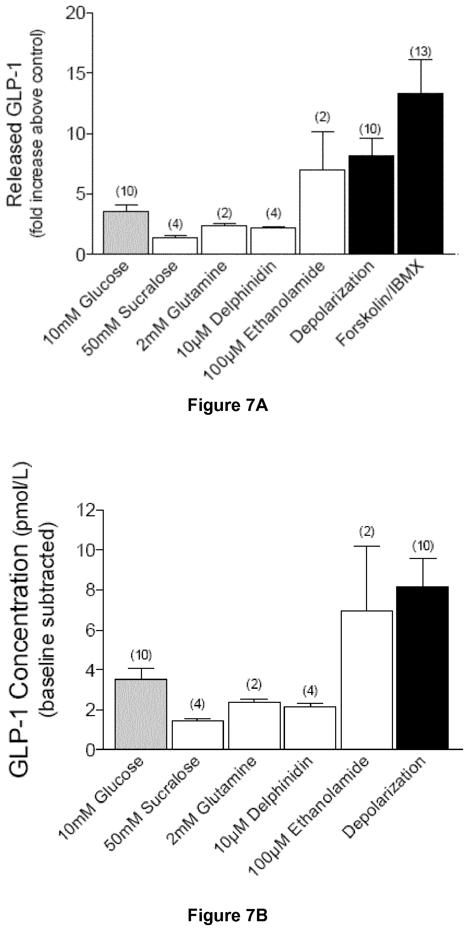

The pharmaceutical oral dosage form of the invention is designed to burst release the active ingredient(s) at the targeted area of the small intestine of a subject, namely the terminal (=distal) jejunum of the, preferably human, subject. For effecting burst release of the core ingredients of the pharmaceutical dosage form, the core contains a disintegrant. Disintegrants for use in the present invention are generally known in the art as rapidly expanding and dissolving when coming into contact with the targeted environment, typically, an aqueous environment as in the present invention, namely, the small intestine of a subject, preferably a human. Disintegrants lead to rapid breakdown of the core of the pharmaceutical oral dosage form of the invention when the core comes into contact of the aqueous medium present in the subject's small intestine. Preferably, the disintegrant in the pharmaceutical dosage form of the invention is selected such that more than 70 % of the core is released within two minutes or less or more than 85 % of the core is released within 5 minutes or less, following contact with water or an aqueous medium like the small intestine of a human subject. A typical time line of a burst release of a pharmaceutical dosage form of the invention is depicted in Fig. 7. Preferred disintegrants in the context of the invention are crosslinked polyvinylpyrrolidones, crosslinked carboxymethyl celluloses and modified starchs. Particularly preferred crosslinked polyvinylpyrrolidones for use in the invention include Polyplasdones, in particular Polyplasdone XL, Polyplasdone XL-10 and Polyplasdone INF-10 (commercially available from Ashland Inc., Covington, KY, USA). Other useful disintegrants include, but are not limited to, polyvinylpolypyrrolidone, croscarmellose and carboxymethylcellulose (preferably, the sodium salt thereof).

The burst release of the core's ingredients, in particular the active compound(s) such as glucose, establishes a steep gradient between intracellular and extracellular levels of the respective active compound(s), preferably glucose, sucralose, ethanolamides, BSA and/or an anthocyanine such as delphinidin 3-rutinoside, in the targeted EEC, preferably L cells, at the site of release of the core ingredients.

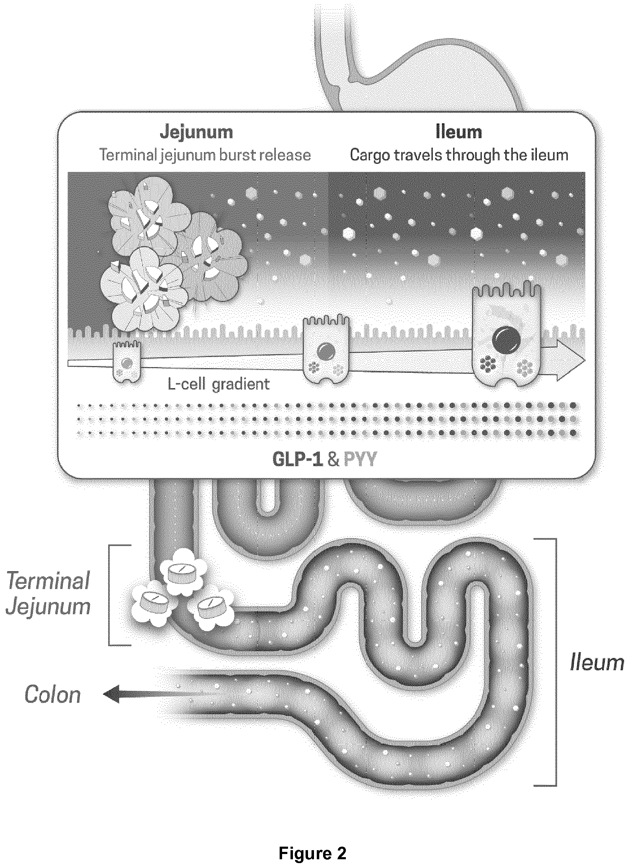

Compared to previous technologies, in particular WO 2010/027498 A2 and WO 2012/118712 A2, the burst release of the active ingredient(s) from the pharmaceutical oral dosage forms of the invention in the terminal jejunum of a subject provides an improved triggering of EECs, in particular L cells, since the compound(s) sensed by the EECs can access the target cells, preferably L cells, over the whole small intestine such that more enterokines, in particular GLP-1 and PYY, are produced and released by the EECs (see also Fig. 2 and its further description herein below). In turn, far less amount of active ingredient(s) such as glucose is needed to be administered to the subject with the aid of the invention as compared to prior art formulations (e.g., 10 g of glucose must be administered to a patient according to WO 2012/118712 A2; cf. Example 1 on page 43 of WO 2012/118712 A2).

The pharmaceutical dosage form of the invention releases its active compound(s) in the terminal jejunum, of a subject, preferably a human subject, through a specific design of a pH sensitive coating. The coating substantially degrades and/or dissolves in the jejunum by specific selection of the enteric coating which is chosen from pH sensitive polymers substantially degrading and/or dissolving at a pH value of about 5.5 to about 7.5, preferably about 7.2 to about 7.3. Such pH sensitive polymers are preferably selected from hydroxypropylmethyl celluloses (also called hereinafter "hypromelloses") and anionic copolymers of methacrylic acid and methacrylmethacrylate. Most preferably, the pH sensitive enteric coating containing or being made of hydroxypropylmethyl cellulose is hydroxypropylmethyl cellulose acetate succinate. A highly preferred commercially available product of this kind is AQOAT®, particularly preferred AQOAT®-HF (Shin-Etsu Chemical Co., Chiyoda, Japan). In other preferred embodiments of the type of anionic copolymers of methacrylic acid and methcrylmethacrylate various forms of Eudragit® polymers may also be used. Eudragit® is commercially available from Evonik Healthcare & Nutrition GmbH, Essen, Germany. In a preferred embodiment, Eudragit® FS30D is used as the pH sensitive polymer of the coating, or at least a part thereof.

In further preferred embodiments of the invention, different coatings can be applied in combination. According to one embodiment, the coating comprises or is made of a combination of a hydroxypropylmethyl cellulose and an anionic copolymer of methacrylic acid and methacrylmethacrylate. Preferably, a combination of coatings is applied such that typically a sub-coating of one pH sensitive polymer is applied as a first layer and a coating of a second pH sensitive polymer is applied on the sub-coating as a second layer. For example, the pH sensitive coating can comprise a sub-coating of or comprising, respectively, a hydroxypropylmethyl cellulose as a first layer, and a second coating comprising or being made of an anionic copolymer of methacrylic acid and methacrylmethacrylate provided as a second layer on the sub-coating. In a further preferred embodiment, the coating of the pharmaceutical oral dosage form of the invention comprises a coating comprising a first layer (sub-coating) comprising or being made of an anionic polymer of methacrylic acid and methacrylmethacrylate such as an Eudragit®, more preferably Eudragit® FS30D, and a second layer comprising or being made of a hydroxypropylmethyl cellulose such as AQOAT®, more preferably AQOAT®-HF. More preferably, the anionic copolymer of methacrylic acid and methacrylmethacrylate, e.g. an Eudragit®, preferably Eurdragit® FS30D, is present in less amount than the hydroxypropylmethyl cellulose such as AQOAT®, more preferably AQOAT®-HF. In other words, the thickness of the first layer of this type of combination is lower then the thickness of the second layer in this combination. More specifically, the ratio of amount or thickness, respectively, between first layer and second layer typically ranges from about 1:10 to about 1:50, particularly preferred from about 1:20 to about 1:30.

For controlling and monitoring the appropriate release and also the spreading of the ingredients of the core of the pharmaceutical oral dosage form of the invention in the intestinal tract of the subject, preferably a human, it is convenient to include a tracer substance in the core part of the pharmaceutical dosage form. A preferred tracer substance is caffeine. One example of a preferred combination of active compound and tracer substance is glucose and caffeine, preferably, about 60 to about 70 % (w/w) glucose and about 2 to about 4 % (w/w) caffeine, most preferred about 67 % (w/w) glucose and about 3.2 % (w/w) caffeine, based on the total weight of the core. The release of the tracer substance from the pharmaceutical oral dosage form in the small intestine can be conveniently monitored by analytical methods generally known in the art. In the case of caffeine, blood samples are taken from the subject before and at suitable time intervals after oral administration of the oral dosage form. After centrifugation of the blood sample, the caffeine content in the serum fraction of the sample is measured by ELISA testing using commercially available test kits (e.g. Caffeine ELISA Kit, BioVision Inc., Milpitas, CA, USA) according to the manufacturer's instructions.

The pharmaceutical oral dosage forms of the invention are preferably fine-tuned in various ways so as to further improve targeted release and triggering of EECs in the small intestine of a subject, preferably a human subject. Moreover, different dosage forms having a variety of release patterns in the subject's small intestine can be combined so as to synergistically act on the subject's EECs and their enterokine output.

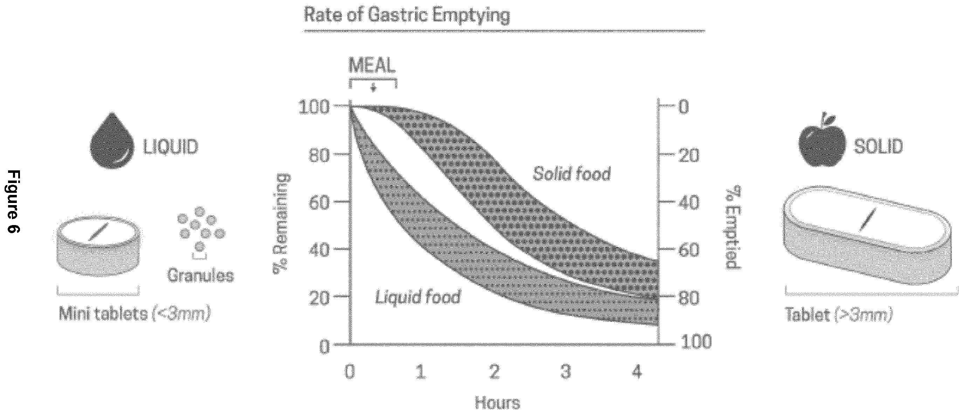

The pharmaceutical dosage forms as outlined above are small in dimension, namely below 3 mm in the largest dimension, more preferably about 0.6 mm to about 1.7 mm in the largest dimension. Such small dosage forms may conveniently take the form of granules or pellets. Small dosage forms of the invention have the benefit of behaving like a fluid in a subject's stomach (cf. Fig. 6) causing a fast and constant entry of the pharmaceutical oral dosage form of the invention into the intestinal tract, and therefore to more evenly transport it to the targeted burst release area in the subject's terminal (i.e. distal part) jejunum.

In other embodiments of the invention, which are not claimed, it may also be convenient that the pharmaceutical oral dosage form is of larger size, i.e. forms wherein the largest dimension of the dosage form is about 3 mm or more, the upper size limit being conveniently selected by the skilled person such that the dosage form can be well swallowed by the subject. A typical range for pharmaceutical oral dosage forms are dosage forms having a largest dimension of about 3 to about 10 mm. It is to be understood that this range includes all integers of mm, namely, 3, 4, 5, 6, 7, 8, 9 and 10 mm as well as any sub-proportions thereof. In addition to the above components the pharmaceutical oral dosage form of the invention may contain further ingredients typically present in oral dosage forms such as tablets, capsules, granules and pellets, for providing and/or improving various parameters. Typical additional ingredients for use in the present invention include excipients, carriers, fillers, glidants, dispersants, plasticizers, wetting agents, anti-tacking agents, neutralization agents and the like. The person skilled in the art of formulating pharmaceutical oral dosage forms is readily able to identify specific compounds and substances of the above and other types as well as their combinations and amounts to be used. Further guidance can be found in Remington's Pharmaceutical Sciences, 18th Ed. Mack Printing Company, 1990, in particular pages 1289-1329.

The pharmaceutical oral dosage form as defined and described herein can be used for the treatment and/or prevention of various conditions, disorders, and/or diseases, particular of human subjects, which relate to the energy household of and metabolism in the, preferably human, body, but also conditions or disorders being the consequence of or relating to, respectively, malfunction of energy household and metabolism. In particular, the pharmaceutical dosage form of the invention is used in the prevention and/or treatment of insulin resistance, type 2 diabetes mellitus, non-alcoholic fatty liver disease, non-alcoholic steatohepatosis, microvascular dysfunction, metabolic syndrome, obesity, cardiovascular diseases, osteoporosis, malabsorption conditions and neurogenerative diseases (such as Alzheimer's Disease) in a subject.

The present invention therefore also relates to a method of prevention and/or treatment of various conditions, disorders, and/or diseases, particularly of human subjects, which relate to the energy household of and metabolism in the, preferably human, body, but also conditions or disorders being the consequence of or relating to, respectively, malfunction of energy household and metabolism, preferably insulin resistance, type 2 diabetes mellitus, non-alcoholic fatty liver disease, non-alcoholic steatohepatosis, microvascular dysfunction, metabolic syndrome, obesity, cardiovascular diseases, osteoporosis or malabsorption conditions, comprising the step of orally administering an effective amount of a pharmaceutical oral dosage form to the subject in need thereof.

A further therapeutic aspect of the invention is based on the known proliferative and antiapoptotic effect of enterokines as described herein, specifically GLP-1 and GLP-2, on cells of the gastro-intestinal tract, especially of the gastro-intestinal mucosa (see, for example, Sigalet (2012) J. Anim. Sci. 90, 1224-1232; Aw et al. (2017) Asia-Pac. J. Clin. Oncol. 14, 23-31; and Kissow et al. (2012) Cancer Chemother. Pharmacol. 70, 39-48). By administration of the pharmaceutical oral dosage forms of the invention it is possible not only to prevent and/treat malabsorption disorders in affected subjects, but also to treat subjects, in particular human subjects, suffering from disorders, diseases and/or conditions of impaired gastro-intestinal function due to irregular gastro-intestinal growth (including reduced intestinal length and impaired/reduced formation of intestinal mucosa and villi, especially, and preferably, in neonatal and infant children, and/or due to disorders of the intestinal mucosa such as mucositis, preferably resulting from chemotherapy, radiotherapy and/or infections, especially in tumour and/or cancer patients. This treatment aspect of the invention also comprises the prevention of the above gastro-intestinal disorders and diseases.

Accordingly, the present invention also relates to the claimed dosage form for use in a method of treatment of the above-described gastro-intestinal conditions comprising the oral administration of an effective amount of the pharmaceutical oral dosage form of the invention to a, preferably human, subject in need thereof, wherein the pharmaceutical oral dosage form preferably leads to the release of GLP-1 and/or GLP-2, in particular a substantial increase of GLP-1 and/or GLP-2 levels, in said subject.

Furthermore, the present invention also relates to the pharmaceutical oral dosage form of the invention for use in the treatment of the above-described gastro-intestinal conditions in a, preferably human, subject in need thereof, wherein the pharmaceutical oral dosage form preferably leads to an increased release of GLP-1 and/or GLP-2, in particular a substantial increase of GLP-1 and/or GLP-2 levels, in said subject.

In general, the administration of the pharmaceutical oral dosage form leads to a substantial increase of the addressed at least one enterokine, preferably GLP-1, GLP-2, GIP, PYY, CCK and/or neurotensin, particularly preferred GLP-1 and/or GLP-2 and/or PYY, above the respective base-line level of the enterokine, in particular the level of the enterokine in the blood serum/plasma of the subject being treated. In preferred embodiments of the invention, the level of the respective enterokine in the subject's blood serum/plasma increases by at least about 50 % or more, preferably about 50 % to about 200 % or even more such as about 300 %, as compared to the level in the blood plasma/serum of the subject before administration of the pharmaceutical oral dosage form of the invention. The increase in enterokine concentration in the subject's blood plasma/serum typically occurs within about 3 to about 6 hours post administration of the pharmaceutical oral dosage form of the invention.

According to the invention, the term "effective amount of the pharmaceutical oral dosage form" depends on various factors such as the specific condition, disorder or disease to be treated and/or prevented, the age and sex of the subject as well as the general condition of the subject. Furthermore, the "effective amount of the pharmaceutical oral dosage form" depends on the type of active compound(s) used. Typically, however, pharmaceutical oral dosage forms of the invention are preferably administered once daily in one or more unit dosages, more preferably in the fasted state of the subject, particularly preferred at least about 30 min, more preferred at least about 45 min, even more preferred at least 1 hour before the first meal of the day. With glucose as a preferred active compound, the effective amount is preferably a daily dose of about 0.5 to about 6 g glucose orally administered in one or more unit dosages in the fasted state of the subject as outlined before. A dose of about 1 to about 5.5 g once daily is more preferred, and about 5 g glucose once daily may be given as a particularly preferred regimen. A typical unit dosage of glucose within the pharmaceutical oral dosage form is about 0.5 g to about 2 g, more preferably about 0.75 to about 1.5 g glucose such as ca. 1 g glucose per unit dosage.

As regards the pharmaceutical oral dosage form, its use and treatment method of the invention as described above, it is of particular benefit when the dosage form is formulated such that the at least one compound stimulating enteroendocrine cells to release an enterokine stimulates said cells present in the intestine of the subject from the jejunum to the ileo-cecal valve of the, preferably human, subject.

The therapeutic use and treatment method of the invention as generally defined above also entail a combination of different pharmaceutical oral dosage forms wherein the dosage forms exhibit different travelling profiles in the subject's gastro-intestinal tract and/or contain different compounds stimulating enteroendocrine cells to release at least one enterokine. For example, one dosage form may contain glucose and further dosage form may contain an anthocyanine (further combinations and specific examples of active compounds and preferred combinations have already been elaborated above in the context of using a single dosage form). A combination of different dosage forms using different active compounds is specifically of benefit when the different compounds are not easily compatible (e.g. as regards their chemical properties) for inclusion in a single dosage form.

In preferred embodiments of this combinatorial approach, the dosage form for use in the treatment method of the invention comprises administering a first pharmaceutical oral dosage form as defined herein having a size of 3 mm or more, preferably 3 to 10 mm, based on the largest dimension of said first dosage form, and administering a second pharmaceutical oral dosage form as defined herein having a size of less than 3 mm, preferably a size of from 0.6 to 1.7 mm, based on the largest dimension of said second dosage form, wherein the active compound in said first and said second oral dosage forms stimulating enteroendocrine cells to release at least one enterokine may be the same or different.

It is, of course, also possible to administer or use, respectively, more than two different sizes and/or more than two different active compounds.

The present invention also relates to pharmaceutical packages comprising two or more different pharmaceutical oral dosage forms according to the invention as outlined above for combinatorial uses and treatment methods as outlined above.

A further aspect of the invention is a method for preparing a pharmaceutical oral dosage form as defined and described herein, the method comprising the steps of:
(a) preparing a mixture comprising at least one nutrient compound stimulating enteroendocrine cells to release at least one enterokine, and at least one disintegrant as defined above,
(b) compressing the mixture obtained in step (a); and
(c) applying to the compressed mixture at least one coating comprising at least one pH sensitive polymer which substantially degrades and/or dissolves at a pH value of about 5.5 to about 7.5.

Optionally, the mixture of step (a) and/or the at least one coating of step (c) may comprise further ingredients as outlined above for the pharmaceutical oral dosage form *per se.* Preferred embodiments of the constituents as defined in steps (a) and (c) have already been described in detail above.

According to a further preferred embodiment of the preparation method of the invention, more than one coating is applied to the compressed mixture obtained in step (b), i.e. the core component of the pharmaceutical oral dosage form of the invention.

In particularly preferred embodiments of the preparation method, a combination of at least two coatings is applied in step (c). Preferably, a sub-coating of one pH sensitive polymer is applied as a first layer and a coating of a second pH sensitive polymer is applied on the sub-coating as a second layer. For example, the pH sensitive coating can comprise a sub-coating of or comprising, respectively, a hydroxypropylmethyl cellulose as a first layer, onto which a second coating comprising or made of an anionic copolymer of methacrylic acid and methacrylmethacrylate as a second layer. In a further preferred embodiment, a first layer (sub-coating) comprising or made of an anionic polymer of methacrylic acid and methacrylmethacrylate such as an Eudragit®, more preferably Eudragit® FS30D, is applied to the core obtained in step (b) and a second layer comprising or made of a hydroxypropylmethyl cellulose such as AQOAT®,more preferably AQOAT®-HF, is applied onto the first layer. More preferably, the anionic copolymer of methacrylic acid and methacrylmethacrylate, e.g. an Eudragit®, preferably Eurdragit® FS30D, is applied in less amount than the the hydroxypropylmethyl cellulose such as AQOAT®, more preferably AQOAT®-HF, of the second layer. In other words, the thickness of the first layer of this type of combination is lower then the thickness of the second layer in this combination. More specifically, the ratio of amount or thickness, respectively, between first layer and second layer typically ranges from about 1:10 to about 1:50, particularly preferred from about 1:20 to about 1:30. In preferred embodiments of the invention, the one or more coatings are applied by spray-coating in step (c).

The Figures show:
Fig. 1: illustrates GLP-1 and PYY release by L cells along the small intestine. The left panel is a schematic representation of the gastro-intestinal tract of a human. The human's small intestine comprises, from gastric side to colon side, the three parts duodenum, jejunum and ileum. L cells are present in the human small intestine from duodenum to ileum with the concentration and maturation of L cells increasing from proximal to distal small intestine. The highest concentration of L cells is found in the terminal (i.e. distal) ileum. Panels on the right show the time dependency of GLP-1 and PYY, respectively, output of L cells in the jejunum (upper right panel) and in the ileum (lower right panel). Upon stimulation by nutrients, here exemplified by glucose, L cells release the enterokines GLP-1 and PYY, whereby GLP-1 is released from L cells throughout the small intestine (i.e. duodenum, jejunum and ileum), however, substantial release of GLP-1 starts in the jejunum and increases from proximal to distal small intestine with peak release in the terminal ileum. On the contrary, PYY release is much lower in the jejunum and increases strongly only in the ileum where GLP-1 and PYY are co-released to substantially equal extend.
Fig. 2: illustrates the improved effect on release of GLP-1 and PYY through the burst release of the enterokine-stimulating content of the pharmaceutical oral dosage forms of the invention in the jejunum, preferably the terminal jejunum. The oral dosage form of the invention specifically releases its core contents in the jejunum of the, preferably human, subject, in a timely sharp manner so that an abrupt bolus of the enterokine-stimulating substance rapidly develops. The enterokine-stimulating substance travels from jejunum to ileum and through ileum making sure that an as large as possible population of L cells is stimulated to release GLP-1 (in the jejunum) and GLP-1 and PYY (in the ileum). In this manner, the pharmaceutical oral dosage form of the invention provides that the at least one compound stimulating enteroendocrine cells to release an enterokine stimulates said cells present in the intestine of the subject from the jejunum to the ileo-cecal valve of the, preferably human, subject. Thus, the strategy according to the invention optimises GLP-1 and PYY release from L cells per unit dose such that the overall dose (per dosage administered and overall dose per time interval such as per day) can be kept low in comparison to prior art approaches.
Fig. 3: shows a schematic representation of a cross-section of a villus in the mucosa of the small intestine of a human subject. GLP-1 is released from L cells in the crypts and on the villi. However, the GLP-1 release capacity per L cells increases with the maturation state of the L cell, i.e. it is highest when the L cells reach the top of the villi. Contrary to GLP-1, L cells start to synthesize PYY only at a higher maturation state. The maturation of L cells can be accelerated by substances such as human milk oligosaccharides (HMO) and a variety of pathway inhibitors (e.g. NOTCH signalling inhibitors). Thus, combining L cell maturation enhancing substances with a nutrient or like glucose or other active compound (such as anthocyanin) in the inventive oral dosage forms leads to a synergistic increase of GLP-1 and PYY by L cells.
Fig. 4: is a schematic representation showing the various molecular mechanisms by which a stimulus to an L cell is translated in the cell to a release of enterokines GLP-1 and PYY. Thus, GLP-1 and PYY are released in response to stimuli that activate glucose transporters (GLUT2, SGLT1) and G protein-coupled receptors such as taste receptors, fatty acid receptors, amino acid receptors and bile acid receptors. The signals generated by the glucose transporters or G-protein-coupled receptors are transmitted by three main mechanisms: transmembrane calcium influx, intracellular calcium release and intracellular cAMP release. Hence, signalling events in L cells can be separated into three segments: (i) glucose transporter (GLUT2 and SGTL1) levels and levels of G protein-coupled receptors; (ii) the intracellular signalling pathway level (calcium and cAMP-dependent pathways); and (iii) the amount of fusion events of vesicles with the plasma membrane to release their content (release level). Through these mechanisms the nature of the released enterokine does not depend on the stimulus or signalling pathways involved but exclusively on (a) the location of the L cell in the small intestine (duodenum, jejunum or ileum) and (b) on the maturation state of the L cell (corresponding to its position in the crypts or on the villi of the mucosa).

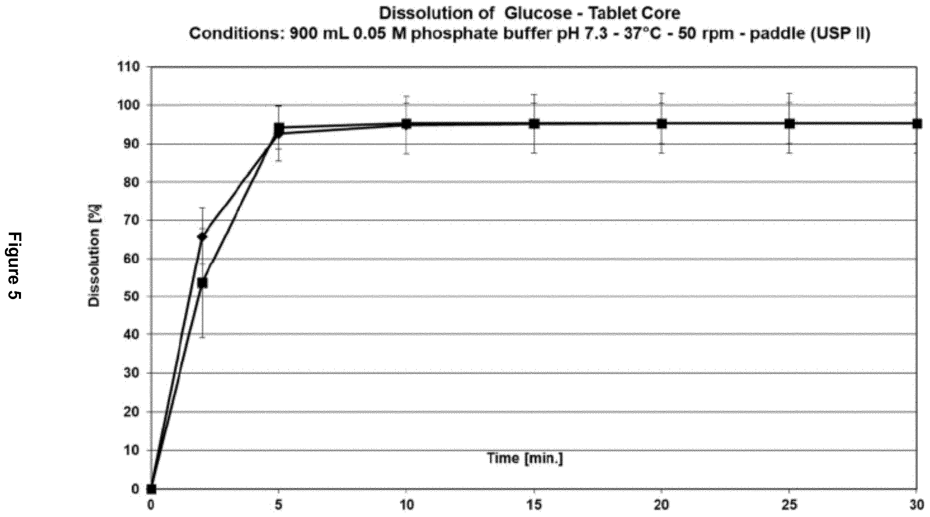

Fig. 5: shows a graphic representation depicting the data of online UV/VIS spectrophotometric measurement of caffeine released from the core of a pharmaceutical oral dosage form of the invention in an aqueous buffer as indicated over time. The data show a burst release of the core components: almost 70 % of the core contents are released into the buffer solution within less than 2 min, and more than 85 % are released within 5 min.
Fig. 6: illustrates the impact of the dimensions of the inventive pharmaceutical dosage form on its behaviour in the gastrointestinal tract: larger oral dosage forms (e.g. tablets, capsules) having a largest dimension of 3 mm or more (upper encircled area in the middle diagram), which are not according to the invention, behave like solid food as regards the rate of gastric emptying. Such larger oral dosage forms show a lag phase after ingestion, slow gastric emptying, they travel slow through the small intestine, and are fractionated into separate boli (in the case of multiple tablets or capsules and the like, fractionation of the overall orally administered dose) through periodic pyloric sphincter opening. On the contrary, small oral dosage forms of less than 3 mm in their largest dimension, which are according to the invention, behave like fluids (lower encircled area in the middle diagram). They show no lag phase in gastric emptying which follows instantaneously after ingestion.
   Consequently, such small oral dosage forms travel much faster through the small intestine as compared to dosage forms having 3 mm or more in their largest dimension, and exhibit only a limited fractionation of the overall dose administered.
Fig. 7: shows graphical representations of experimental data obtained from GLUTag cells (as a cell culture model of human L cells, especially in terms of their stimulation properties to secrete enterokines; cf. Kuhre et al. (2016) J. Mol. Endocrinol. 56 (3), 201-211) stimulated by the indicated active compounds at the indicated concentrations (depolarization and the mixture of Forskolin (3*R*,4a*R*,5*S*,6*S*,6a*S*,10*S*,10a*R*,10b*S*)-dodecahydro-5,6,10,10b-tetrahydroxy-3,4a,7,7,10a-pentamethyl-1-oxo-3-vinyl-1*H*-naphtho[2,1-*b*]pyran-5-yl acetat)/BMK (the venom of scorpion *Buthus martensii* Karsch) were used as positive controls). GLP-1 release was measured in the stimulation medium supernatant. (A) Data of GLP-1 release are expressed as fold increase in comparison to negative control (stimulation medium supernatant of cells without treatment by any stimulus) (B) Data of GLP-1 release are expressed as GLP-1 concentration in the stimulation medium supernatant with signal of negative control (stimulation medium supernatant of cells without treatment by any stimulus) subtracted. Numbers in brackets above the columns indicate the number of independent experiment for each stimulant or control, respectively.

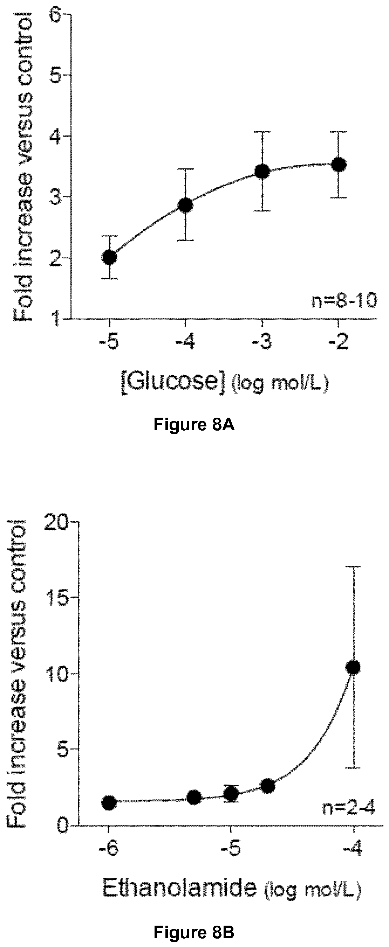

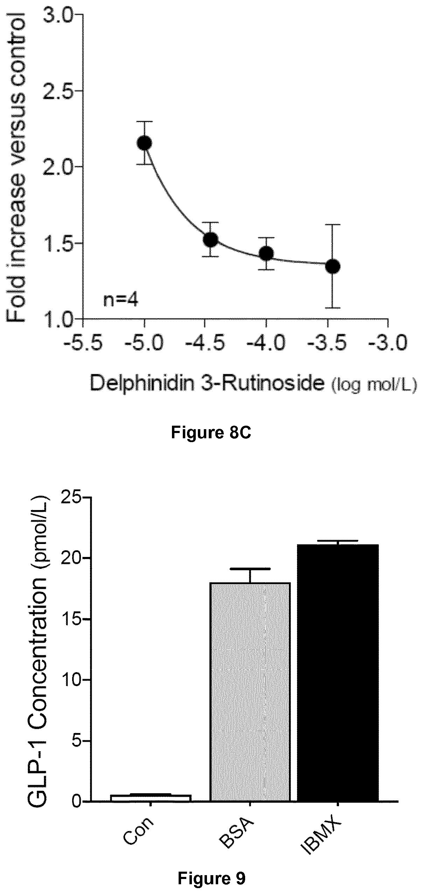

Fig. 8: shows graphical representations of dose response data of GLUTag cells stimulated with glucose (Fig. 8A), ethanolamide (Fig. 8B) or delphinidin 3-rutinoside (Fig. 8C) at the indicated concentrations.
Fig. 9: shows a graphical representation of experimental data obtained from GLUTag cells (as a cell culture model of L cells) stimulated by BSA at 0.5 % (w/v). GLP-1 concentration was measured in the stimulation medium supernatant. The phosphodiesterase inhibitor 3-isobutyl-1-methylxanthine (IBMX) was used as positive control. Negative control was stimulation medium supernatant only.

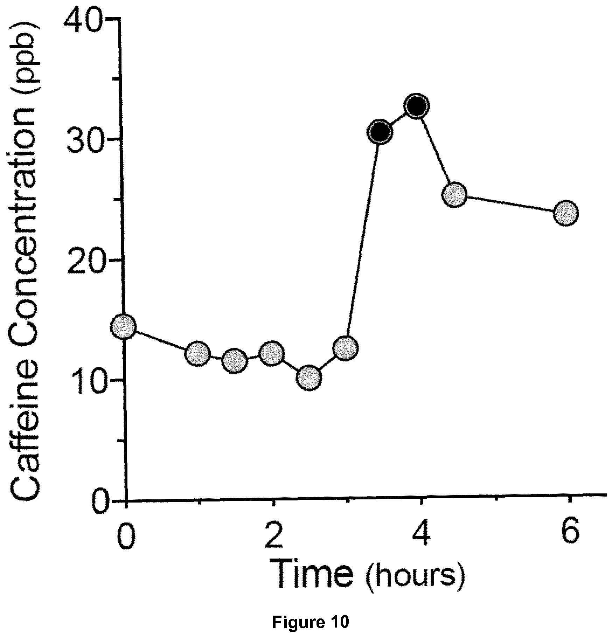

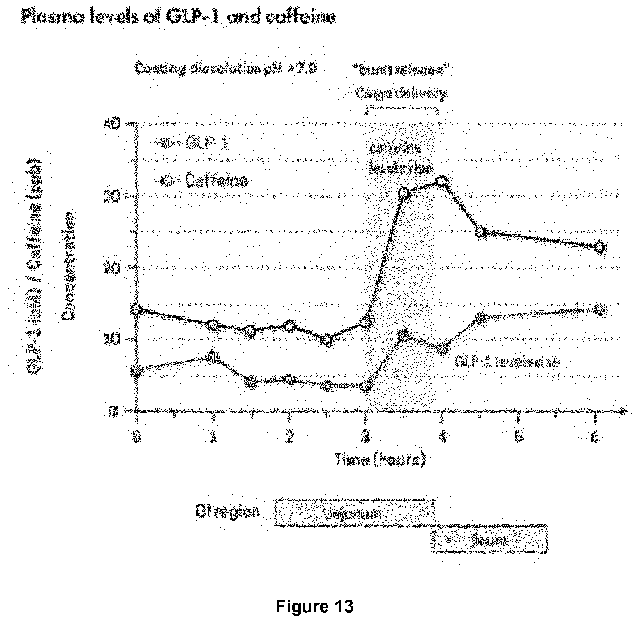

Fig. 10: shows a graphical representation of experimental *in vivo* data of caffeine concentrations in blood serum taken before and at the indicated time points after ingestion of a pharmaceutical oral dosage form of the invention corresponding to 125 mg glucose administered orally to a volunteer after overnight fasting. The data show a sharp increase of caffeine concentration from 3 to 4 hours post administration of the pharmaceutical oral dosage form. The data confirm the burst release behaviour of the pharmaceutical oral dosage form of the invention. The onset of the peak interval at 3 hours post administration indicates that the release occurs in the terminal jejunum of the volunteer; cf. also Fig. 13.

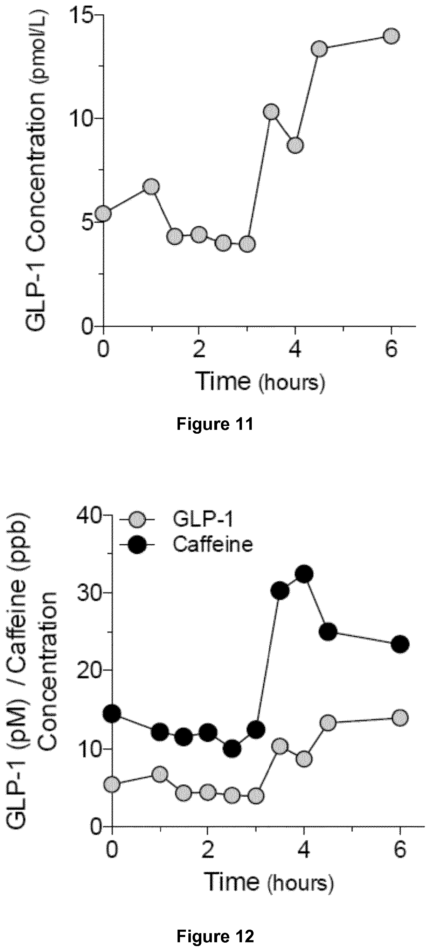

Fig. 11: shows a graphical representation of experimental *in vivo* data of GLP-1 concentrations in blood serum taken as described above for Fig. 10. From a GLP-1 baseline concentration of 5 pmol/I measured before administration of the pharmaceutical oral dosage form of the invention administered in a total dose of 125 mg glucose, the GLP-1 concentration doubles from 3 to 4 hours post administration, and reaches a plateau concentration of almost 15 pmol/I serum.
Fig. 12: shows an overlay of the data of Fig. 10 (caffeine serum concentration) and Fig. 11 (GLP-1 serum concentration) indicating that sharp increase in GLP-1 concentration matches the burst release of the components from the core of the pharmaceutical oral dosage form of the invention.
Fig. 13: shows a further overlay of the caffeine and GLP-1 concentration, respectively, according to Fig. 12, but showing also the time post administration which corresponds to a localization of the pharmaceutical oral dosage from in the jejunum and ileum, respectively (indicated below the horizontal axis), according to known data of travelling times of typical pharmaceutical oral dosage forms as exemplified by a pH-sensitive and radiotelemetry capsule disclosed in Evans et al. (1988).

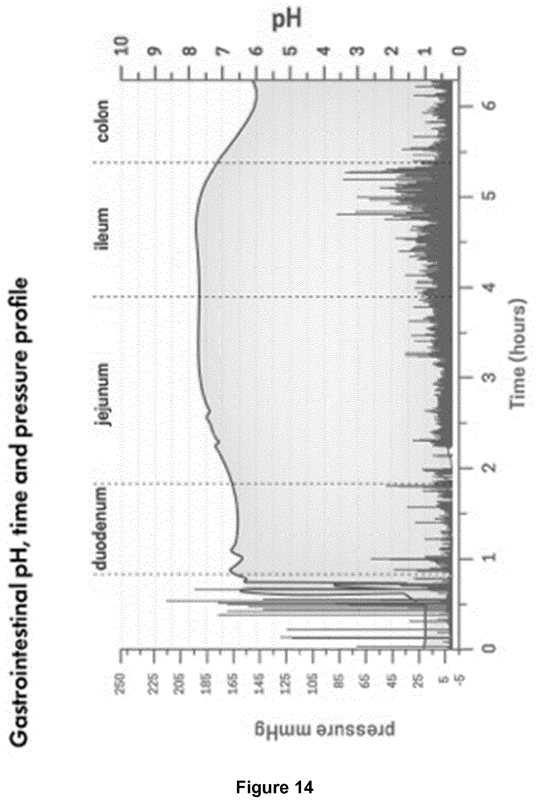

Fig. 14: shows a graphical representation of data of travelling time, localization and measured pH of a pH sensitive radiotelemetry capsule as measured by Evans et al. (1988) Gut 29, 1035-1041. The Fig. is a reproduction of Fig. 3 of Evans et al. (1988), page 1038.

The following non-limiting examples further illustrate the present invention.

### EXAMPLES

### Example 1: Burst release tablet core

A core for a pharmaceutical oral dosage form of the invention has been developed to provide a burst release kinetic of disintegration in tap water or aqueous buffer of less than 2 min. Glucose has been selected as an example of the active compound.

A glucose gentle direct compression process was used employing specific functional excipients to enable a good followability as well as processability and tablet properties on the one hand side and a fast disintegration and dissolution behaviour of glucose on the other hand. More specifically, the following excipients were selected:
- Silicified microcrystalline cellulose (Prosolv SMCC 90) has been selected and utilized as a binder..
- Crossinked povidone - Crospovidone (Polyplasdone XL-10) has been selected as super-disintegrant to provide a burst release kinetic.
- Magnesium stearate and colloidal silicon dioxide (Aerosil 200) have been selected as lubricant and glidant agents, respectively.

The final composition of the tablet core is given in Table 1

**Tab. 1. Composition of the glucose tablet core (reference example)**

| Compound | Function | Concentration [% (w/w)] |
|---|---|---|
| Glucose anhydrous | Active Pharmaceutical Ingredient (API) | 60.0 |
| Caffeine | Internal standard | 1.4 |
| Polyplasonde XL-10 | Disintegrant | 5.0 |
| Magnestium stearate | Lubricant | 1.0 |
| Aerosil 200 | Glidant | 0.2 |
| Prosolv SMCC 90 | Binder | 32.4 |
| Sum | | 100.0 |

The prepared oral dosage form showed the following general characteristics:
Weight: 1.67 +/- 1.2 %
Height: 8.3 mm +/- 0.4 %
Breaking strength: 245 N +/- 7 %
Disintegration: < 30 s

The burst release of the tablet core in aqueous buffer (500 ml 0.05 M phosphate buffer, pH 7.3 at 37 °C) was measured by online UV/VIS spectrophotometry of the caffeine as internal standard. The results of two independent experiments are shown in Fig. 5. The results show a fast dissolution of about > 85 % release of the internal standard within 5 min.

### Example 2: Hypromellose acetate succinate-coated tablet (reference example)

The tablet core of Example 1 was coated with AQOAT®-HF to provide an enteric coating and drug please at a specific pH value of >6.8, specifically at pH 7.3. In more detail, the tablet core of Example 1 was spray-coated using the following coating composition:

**Tab. 2:AQOATO coating composition**

| Compound | Function | Concentration [% (w/w)] |
|---|---|---|
| Water | Dispersant | 91.9 |
| Triethyl citrate (TEC) | Plasticizer | 1.7 |
| Sodium lauryl sulfate (SLS) | Wetting agent | 0.3 |
| Talcum | Anti-tacking agent | 1.4 |
| AQOAT® AS-H F | pH sensitive polymer | 4.7 |
| Sum | | 100.0 |

The above coating was applied on the tablet core of Example 1 by using a LCH 25 Lödige lab-coater (Gebrüder Lödige Maschinenbau GmbH, Paderborn, Germany).

### Example 3: Eudragit®-coated tablet (reference example)

In an alternative embodiment of the invention, the tablet core of Example 1 was coated with Eudragit® FS30D to provide an enteric coating and drug release at a pH value of >7.0, specifically at pH 7.3. In more detail, the tablet core of Example 1 was spray-coated using the following coating composition:

**Tab. 3: Eudragit® coating composition**

| Compound | Function | Concentration [% (w/w)] |
|---|---|---|
| Water | Dispersant | 80.0 |
| Plasacryl (CMS TEC Polysorbate 80) | Anti-tacking, plasticizer, wetting agent | 1.8 |
| Eudragit® FS30D | pH-sensitive polymer | 18.2 |
| Sum | | 100.0 |

The above coating was applied on the tablet core of Example 1 by using a LCH 25 Lödige lab-coater (Gebrüder Lödige Maschinenbau GmbH, Paderborn, Germany).

### Example 4: GLP-1 release of GLUTag cells in response to different active compounds

Murine GLUTag cells (ATCC, Manassas, VA, USA) of 80 % confluency were split from a T25 into 6 wells of a 12-well plate. On day 1, the resulting cells were 40-50% confluent and on day 2 the cells were 50-60% confluent. The cells were mostly single cells or very small aggregates; with no large aggregates observable.

Cells were gently washed 2 times with warm PBS (supplemented with calcium; D8662). Then, 300 µl of stimulation medium, either non-supplemented PBS as negative control or PBS supplemented with 10µM FSK/IBMX (positive control), 10 mM glucose, 50 mM sucralose, 10 µM delphinidin 3-rutinoside or 100 µM oleoylethanolamide, were added to the cells. The cells were then incubated for 2 hours at 37°C.

The 300 µl of stimulation medium were removed from the well and centrifuged for 5 minutes. 200 µl of the supernatant were transferred to a fresh test tube and the samples were stored at -80°C until subjected to GLP-1 measurement. The cells were discarded.

GLP-1 in the supernatant was measured using a commercially available ELISA (Mouse GLP-1 Elisa Kit, Chrystal Chem (Europe), Zaandam, The Netherlands) according to the manufacturer's instructions.

The results of this experiment are shown in Fig. 7 (A: data expressed as fold increase of measured GLP-1 concentration in the sample over negative control; B: data expressed as GLP-1 concentration in the supernatant after subtraction of signal measured in negative control).

In a further experiment, dose-response data of the stimulants glucose (at concentrations of 0,01 mM, 0,1 mM, 1 mM and 10 mM), oleoylethanolamide (at concentrations of 1 µM, 10 µM and 100 µM) and delphinidin 3-rutinoside (at concentrations of 10 µM, 50 µM, 100 µM and 500 µM) were measured as described above. The results are shown in Fig. 8A (glucose), 8B (ethanolamide) and 8C (delphinidin 3-rutinoside), respectively.

In another experiment, BSA (at a concentration of 0.5 % (w/v)) was used as a peptidic stimulant of GLP-1 release by GLUTag cells. Again, stimulation medium alone (PBS) was used as negative control, and IBMX served as the positive control. The results are shown in Fig. 9.

### Example 5: Burst release of components from the non-inventive pharmaceutical oral dosage form in vivo

In order to confirm that the components of the core of the pharmaceutical oral dosage form are released at the appropriate site in the small intestine, the following *in vivo* experiment was carried out:
A healthy volunteer was administered orally with a total of 5 g glucose using 5 units of the oral dosage form according to Example 3, with each tablet containing 1 g glucose. As described in Example 3, the unit dosages contained caffeine as tracer substance (25 mg caffeine per unit dose, 125 mg total amount administered). The administration of the oral dosage forms was carried out in the fasted state after overnight fastening and abstaining 48 hours before the administration of the oral dosage forms from any intake of caffeine-containing food or beverages.

Blood samples were collected from the volunteer before and 1 hour after administration of the oral dosage forms, and then continuing every 30 min until 6 hours post administration of the oral dosage forms. Blood samples were collected in P800 metabolic sample vacutainers (Becton Dickinson Co., Franklin Lakes, NJ, USA), immediately centrifuged (2000 rcf for 10 minutes at 4°C), and the resulting supernatant plasma/serum was then transferred to a fresh tube and frozen on dry ice. Samples were stored at -150°C until measurement of caffeine using a Caffeine ELISA Kit (BioVision Inc., Milpitas, CA, USA) according to the manufacturer's instructions. The caffeine concentration in the blood samples were calculated by comparison to measured samples of caffeine standard concentrations included in the test kit.

The results are shown in Fig. 10 demonstrate a sharp increase in blood caffeine concentration starting at 3 hours post administration of the pharmaceutical oral dosage form of the invention corresponding to a burst release of the pharmaceutical oral dosage form in the terminal jejunum of the volunteer; cf. also Fig. 13 and Fig. 14.

### Example 6: (for reference) The pharmaceutical oral dosage form leads to a pronounced increase of GLP-1 serum concentration following burst release in the jejunum of a subject

Parallel to the measurement of the tracer substance caffeine, GLP-1 concentrations were measured in the serum/plasma samples obtained from the volunteer according to Example 5. GLP-1 concentrations were measured using a commercially available ELISA test (GLP-1 7-36a Human ELISA Kit, ThermoFisher Corp., Waltham, MA, USA) according to the manufacturer's instructions.

The results are shown in Fig. 11. The GLP-1 concentration raises sharply from 5 pmol/I to 10 pmol/I from 3 to 4 hours post administration of 5 pharmaceutical oral dosage forms of the invention each containing 1 g glucose (total dose of 5 g glucose). As the released active compound travels through the small intestine it reaches and triggers further L cells present in the downstream part (ileum) such that the GLP-1 concentration further increases to a value of almost 15 pmol/I after 6 hours post administration.

As shown in Fig. 12 and Fig. 13, the increase in GLP-1 concentration matches the burst release of the components of the pharmaceutical oral dosage form of the invention as measured by the caffeine concentration in the volunteer's blood serum.

From Evans et al. (1988) Gut 29, 1035-1041, the travelling times of pharmaceutical dosage forms of the type of dosage forms of the invention and pH distribution in the various parts of the human intestinal tract, and in particular of the small intestine, are known (see, in particular Fig. 3 on page 1038 of Evans et al. (1988); reproduced in present Fig. 14). In particular, the localization of the pH sensitive radiotelemetry capsule post oral intake have been assessed by Evans et al. (1988) as shown below in Table 4:

**Tab. 4: Localisation of pH sensitive telemetry capsule in the gastro-intestinal tract (GI) in dependency of time after intake (according to Evans et al. (1988) Gut 29, 1035-1041)**

| **GI region** | Time (hours) after intake |
|---|---|
| Duodenum | 0.75 to 1.8 |
| Jejunum | 1.8 to 3.8 |
| Ileum | 3.8 to 5.4 |
| Colon | ≥ 5.4 |

Applying the data of Evans et al. (1988), the burst release of the components of the core of the pharmaceutical dosage form of the invention occurred in the (terminal) jejunum of the subject at a pH environment of > pH 7.0, and in particular ca. 7.3; cf. Fig. 13 and Fig. 14. The *in vivo* data demonstrate that a comparatively low dose of 5 g glucose administered using the pharmaceutical oral dosage form is sufficient to markedly increase the GLP-1 serum concentration in humans. This effect can be likely attributed to the burst release of the core components from the pharmaceutical oral dosage form in the jejunum of the subject.

## Claims

1. Pharmaceutical oral dosage forms comprising a core and a pH-sensitive enteric coating, wherein the core comprises at least one nutrient compound stimulating enteroendocrine cells to release at least one enterokine, and at least one disintegrant providing a burst release of the ingredients of the core when the coating is substantially degraded and/or dissolved, and wherein the coating comprises a pH sensitive polymer which substantially degrades and/or dissolves at a pH value of about 5.5 to about 7.5, wherein the dosage forms have a size of less than 3 mm, based on the largest dimension of the oral dosage forms, and release said nutrient compound in the terminal jejunum of a subject.

2. The oral dosage forms of claim 1 wherein the at least one compound is selected from the group consisting of compounds stimulating the enteroendocrine cells by a mechanism selected from the group consisting of transport into enteroendocrine cells by a transporter expressed by said cells wherein the transporter is selected from the group consisting of GLUT2 and SGLT1, and binding to G protein-coupled receptors expressed by said cells.

3. The oral dosage forms of claim 2 wherein the G-protein-coupled receptor is selected from bile acid receptors, amino acid receptors, peptide receptors and fatty acid receptors and taste receptors.

4. The oral dosage forms of claim 2 or 3 wherein said compound(s) is/are selected from the group consisting of carbohydrates, fatty acids, bile acids, peptides, amino acids, alcohol amides, and anthocyanins.

5. The oral dosage forms of claim 4 wherein the core contains glucose, and optionally one or more compounds selected from sucralose, fatty acids having 2 to 6 carbon atoms, oleic acid, bile acids, peptides, amino acids, ethanolamides and anthocyanins.

6. The oral dosage forms of claim 4 or 5 wherein the anthocyanin is delphinidin 3-rutinoside.

7. The oral dosage forms according to any one of the preceding claims wherein the enteroendocrine cells are selected from the group consisting of I cells, K cells and L cells.

8. The oral dosage forms according to any one of the preceding claims wherein the core further contains an enteroendocrine cell maturation agent.

9. The oral dosage forms of claim 8 wherein the maturation agent is a human milk oligosaccharide (HMO).

10. The oral dosage forms according to any one of the preceding claims wherein the pH sensitive polymer substantially degrades and/or dissolves at a pH value of about 7.2 to about 7.3.

11. The oral dosage forms according to any one of the preceding claims wherein the pH sensitive polymer is selected from the group consisting of hydroxypropylmethyl celluloses and anionic copolymers of methacrylic acid and methacrylmethacrylate.

12. The oral dosage forms of claim 11 wherein the hydroxypropylmethyl cellulose is hydroxypropylmethyl cellulose acetate succinate.

13. The oral dosage forms of claim 11 or 12 wherein the coating comprises a first and a second layer of a pH sensitive polymer, the second layer being coated onto the first layer.

14. The oral dosage forms of claim 13 wherein the first layer contains or is made of an anionic copolymer of methacrylic acid and methacrylmethacrylate.

15. The oral dosage forms of claim 13 or 14 wherein the second layer contains or is made of hydroxypropylmethyl cellulose.

16. The oral dosage forms according to any one of claims 13 to 15 wherein the ratio of thickness between the first and the second layer is from about 1:10 to about 1:50.

17. The oral dosage forms according to any one of the preceding claims wherein the disintegrant is selected from the group consisting of a crosslinked polyvinylpyrrolidone, a crosslinked carboxymethyl cellulose and a modified starch.

18. The oral dosage forms of claim 17 wherein the crosslinked polyvinylpyrrolidone is Polyplasdone.

19. The oral dosage forms according to any one of the preceding claims wherein the enterokine selected from the group consisting of GLP-1, PYY, GLP-2, CCK, GIP and neurotensin, preferably GLP-1 and PYY.

20. The oral dosage forms according to any one of the preceding claims wherein the core comprises a tracer substance.

21. The oral dosage forms of claim 20 wherein the tracer substance is caffeine.

22. The oral dosage forms of claim 21 wherein the core contains 60 to 70 % (w/w) glucose and 2 to 4 % (w/w) caffeine, based on the total weight of the core.

23. The oral dosage forms of claim 22 wherein the core contains 67 % (w/w) glucose and 3.2 % (w/w) caffeine, based on the total weight of the core.

24. The oral dosage forms according to any one of the preceding claims being in the form of a tablet, capsule, pellet or granule.

25. The oral dosage forms according to any one of the preceding claims wherein the size is from about 0.6 mm to about 1.7 mm, based on the largest dimension of the oral dosage form.

26. Pharmaceutical oral dosage forms according to any one of the preceding claims for use in the prevention and/or treatment of a condition or disease selected from the group consisting of insulin resistance, type 2 diabetes mellitus, non-alcoholic fatty liver disease, non-alcoholic steatohepatosis, microvascular dysfunction, metabolic syndrome, obesity, cardiovascular diseases, osteoporosis, neurodegenerative diseases, impaired gastro-intestinal function and malabsorption conditions in a subject.

27. The pharmaceutical oral dosage forms for use of claim 26 wherein said dosage forms are formulated such that the at least one compound stimulating enteroendocrine cells to release an enterokine stimulates said cells present in the intestine of the subject from the terminal jejunum to the ileo-cecal valve of the subject.

28. A method for preparing pharmaceutical oral dosage forms according to any one of claims 1 to 25 comprising the steps of:
(a) preparing a mixture comprising at least one nutrient compound stimulating enteroendocrine cells to release at least one enterokine, and at least one disintegrant;
(b) compressing the mixture obtained in step (a); and
(c) applying to the compressed mixture at least one coating comprising at least one pH sensitive polymer which substantially degrades and/or dissolves at a pH value of about 5.5 to about 7.5.

## Patentansprüche

1. Pharmazeutische orale Darreichungsformen, die einen Kern und eine pH-sensitive enterische Beschichtung umfassen, wobei der Kern mindestens eine Nährstoffverbindung, die enteroendokrine Zellen zur Freisetzung mindestens eines Enterokins stimuliert, und mindestens ein Sprengmittel umfasst, das eine Sprengfreisetzung der Bestandteile des Kerns erzeugt, wenn die Beschichtung im Wesentlichen abgebaut und/oder aufgelöst ist, und wobei die Beschichtung ein pH-sensitives Polymer umfasst, das bei einem pH-Wert von etwa 5,5 bis etwa 7,5 im Wesentlichen abgebaut und/oder aufgelöst wird, wobei die Darreichungsformen eine Größe von weniger als 3 mm, bezogen auf die größte Dimension der oralen Darreichungsformen, aufweisen und die Nährstoffverbindung im terminalen Jejunum eines Subjekts freisetzen.

2. Orale Darreichungsformen nach Anspruch 1, wobei die mindestens eine Verbindung ausgewählt ist aus der Gruppe, bestehend aus Verbindungen, die enteroendokrine Zellen durch einen Mechanismus stimulieren, der aus der Gruppe, bestehend aus dem Transport in enteroendokrine Zellen durch einen Transporter, der von den Zellen exprimiert wird, wobei der Transporter aus der Gruppe ausgewählt ist, die aus GLUT2 und SGLT1 besteht, und Bindung an G-Protein- gekoppelte Rezeptoren, die durch die Zellen exprimiert werden, ausgewählt ist.

3. Orale Darreichungsformen nach Anspruch 2, wobei der G-Protein-gekoppelte Rezeptor aus Gallensäurerezeptoren, Aminosäurerezeptoren, Peptidrezeptoren und Fettsäurerezeptoren und Geschmacksrezeptoren ausgewählt ist.

4. Orale Darreichungsformen nach Anspruch 2 oder 3, wobei die Verbindung(en) aus der Gruppe, bestehend aus Kohlenhydraten, Fettsäuren, Gallensäuren, Peptiden, Aminosäuren, Alkoholamiden und Anthocyaninen, ausgewählt ist/sind.

5. Orale Darreichungsformen nach Anspruch 4, wobei der Kern Glucose und gegebenenfalls eine oder mehrere Verbindungen enthält, die aus Sucralose, Fettsäuren mit 2 bis 6 Kohlenstoffatomen, Oleinsäure, Gallensäuren, Peptiden, Aminosäuren, Ethanolamiden und Anthocyaninen, ausgewählt ist/sind.

6. Orale Darreichungsformen nach Anspruch 4 oder 5, wobei das Anthocyanin Delphinidin-3-rutinosid ist.

7. Orale Darreichungsformen nach einem der vorhergehenden Ansprüche, wobei die enteroendokrinen Zellen aus der Gruppe, bestehend aus I-Zellen, K-Zellen und L-Zellen, ausgewählt sind.

8. Orale Darreichungsformen nach einem der vorhergehenden Ansprüche, wobei der Kern außerdem ein Reifungsmittel für enteroendokrine Zellen enthält.

9. Orale Darreichungsformen nach Anspruch 8, wobei das Reifungsmittel ein humanes Milcholigosaccharid (HMO) ist.

10. Orale Darreichungsformen nach einem der vorherigen Ansprüche, wobei das pH-sensitive Polymer bei einem pH-Wert von etwa 7,2 bis etwa 7,3 im Wesentlichen abgebaut und/oder aufgelöst wird.

11. Orale Darreichungsformen nach einem der vorherigen Ansprüche, wobei das pH-sensitive Polymer aus der Gruppe, bestehend aus Hydroxypropylmethylcellulosen und anionischen Copolymeren von Methacrylsäure und Methacrylmethacrylat, ausgewählt ist.

12. Orale Darreichungsformen nach Anspruch 11, wobei die Hydroxypropylmethylcellulose Hydroxypropylmethylcelluloseacetatsuccinat ist.

13. Orale Darreichungsformen nach Anspruch 11 oder 12, wobei die Beschichtung eine erste und eine zweite Schicht eines pH-sensitiven Polymers umfasst, wobei die zweite Schicht auf die erste Schicht aufgebracht ist.

14. Orale Darreichungsformen nach Anspruch 13, wobei die erste Schicht ein anionisches Copolymer von Methacrylsäure und Methacrylmethacrylat enthält oder daraus besteht.

15. Orlae Darreichungsformen nach Anspruch 13 oder 14, wobei die zweite Schicht Hydroxypropylmethylcellulose enthält oder daraus besteht.

16. Orale Darreichungsformen nach einem der Ansprüche 13 bis 15, wobei das Verhältnis der Dicke zwischen der ersten und der zweiten Schicht etwa 1:10 bis etwa 1:50 beträgt.

17. Orale Darreichungsformen nach einem der vorhergehenden Ansprüche, wobei das Sprengmittel aus der Gruppe, bestehend aus einem quervernetzten Polyvinylpyrrolidon, einer quervernetzten Carboxymethylcellulose und einer modifizierten Stärke, ausgewählt ist.

18. Orale Darreichungsformen nach Anspruch 17, wobei das quervernetzte Polyvinylpyrrolidon Polyplasdon ist.

19. Orale Darreichungsformen nach einem der vorherigen Ansprüche, wobei das Enterokin aus der Gruppe, bestehend aus GLP-1, PYY, GLP-2, CCK, GIP und Neurotensin, vorzugsweise GLP-1 und PYY, ausgewählt ist.

20. Orale Darreichungsformen nach einem der vorherigen Ansprüche, wobei der Kern eine Tracersubstanz umfasst.

21. Orale Darreichungsformen nach Anspruch 20, wobei die Tracersubstanz Coffein ist.

22. Orale Darreichungsformen nach Anspruch 21, wobei der Kern 60 bis 70 % (w/w) Glucose und 2 bis 4 % (w/w) Koffein, bezogen auf das Gesamtgewicht des Kerns, enthält.

23. Orale Darreichungsformen nach Anspruch 22, wobei der Kern 67 % (w/w) Glucose und 3,2 % (w/w) Koffein, bezogen auf das Gesamtgewicht des Kerns, enthält.

24. Orale Darreichungsformen nach einem der vorherigen Ansprüche in Form einer Tablette, einer Kapsel, von Pellets oder eines Granulats.

25. Orale Darreichungsformen nach einem der vorherigen Ansprüche, wobei die Größe von etwa 0,6 mm bis etwa 1,7 mm, bezogen auf die größte Dimension der oralen Dosierungsform, beträgt.

26. Pharmazeutische orale Darreichungsformen nach einem der vorherigen Ansprüche zur Verwendung in der Verhinderung und/oder Behandlung eines Zustands oder einer Erkrankung in einem Subjekt, der/die aus der Gruppe, bestehend aus Insulinresistenz, Typ 2 Diabetes mellitus, nichtalkoholische Fettleber, nichtalkoholische Steatohepatose, Mikrovaskuläre Dysfunktion, Metabolisches Syndrom, Fettleibigkeit, kardiovaskuläre Erkrankungen, Osteoporose, neurodegenerative Erkrankungen, gestörter gastrointestinaler Funktion und Malabsorptionszuständen, ausgewählt ist.

27. Pharmazeutische orale Darreichungsformen zur Verwendung nach Anspruch 26, wobei die Darreichungsformen derart formuliert sind, dass die mindestens eine Verbindung, die enteroendokrine Zellen zur Freisetzung eines Enterokins stimuliert, die im Darm des Subjekts vom terminalen Jejunum bis zur ileo-zäkal-Klappe des Subjekts vorhandenen besagten Zellen stimuliert.

28. Verfahren zur Herstellung pharmazeutischer oraler Darreichungsformen nach einem der Ansprüche 1 bis 25, das die Schritte
(a) Herstellen eines Gemischs, das mindestens eine Nährstoffverbindung, die enteroendokrine Zellen zur Freisetzung mindestens eines Enterokins stimuliert, und mindestens ein Sprengmittel, umfasst,
(b) Komprimieren des im Schritt (a) erhaltenen Gemischs und
(c) Auftragen mindestens einer Beschichtung, die mindestens ein pH-sensitives Polymer umfasst, das bei einem pH-Wert von etwa 5,5 bis etwa 7,5 abgebaut und/oder aufgelöst wird, auf das komprimierte Gemisch umfasst.

## Revendications

1. Formes posologiques orales pharmaceutiques comprenant un noyau et un enrobage entérique sensible au pH, dans lesquelles le noyau comprend au moins un composé nutritif stimulant des cellules entéroendocrines pour libérer au moins une entérokine, et au moins un délitant fournissant une libération en rafale des ingrédients du noyau lorsque l'enrobage est sensiblement dégradé et/ou dissous, et dans lesquelles l'enrobage comprend un polymère sensible au pH qui se dégrade et/ou se dissout sensiblement à une valeur de pH d'environ 5,5 à environ 7,5, dans lesquelles les formes posologiques ont une taille inférieure à 3 mm, sur la base de la plus grande dimension des formes posologiques orales, et libèrent ledit composé nutritif dans le jéjunum terminal d'un sujet.

2. Formes posologiques orales selon la revendication 1, dans lesquelles le au moins un composé est choisi dans le groupe comprenant des composés stimulant les cellules entéroendocrines par un mécanisme choisi dans le groupe comprenant un transport dans des cellules entéroendocrines par un transporteur exprimé par lesdites cellules, le transporteur étant choisi dans le groupe comprenant GLUT2 et SGLT1, et se liant à des récepteurs couplés à la protéine G exprimés par lesdites cellules.

3. Formes posologiques orales selon la revendication 2, dans lesquelles le récepteur couplé à la protéine G est choisi parmi des récepteurs d'acides biliaires, des récepteurs d'acides aminés, des récepteurs de peptides et des récepteurs d'acides gras et des récepteurs de goût.

4. Formes posologiques orales selon la revendication 2 ou 3, dans lesquelles ledit (lesdits) composé(s) est (sont) choisi (s) dans le groupe constitué par des hydrates de carbone, des acides gras, des acides biliaires, des peptides, des acides aminés, des amides d'alcool et des anthocyanines.

5. Formes posologiques orales selon la revendication 4, dans lesquelles le noyau contient du glucose, et facultativement un ou plusieurs composés choisis parmi le sucralose, des acides gras ayant 2 à 6 atomes de carbone, de l'acide oléique, des acides biliaires, des peptides, des acides aminés, des éthanolamides et des anthocyanines.

6. Formes posologiques orales selon la revendication 4 ou 5, dans lesquelles l'anthocyanine est le delphinidine 3-rutinoside.

7. Formes posologiques orales selon l'une quelconque des revendications précédentes, dans lesquelles les cellules entéroendocrines sont choisies dans le groupe constitué par des cellules I, des cellules K et des cellules L.

8. Formes posologiques orales selon l'une quelconque des revendications précédentes, dans lesquelles le noyau contient en outre un agent de maturation de cellules entéroendocrines.

9. Formes posologiques orales selon la revendication 8, dans lesquelles l'agent de maturation est un oligosaccharide de lait humain (HMO).

10. Formes posologiques orales selon l'une quelconque des revendications précédentes, dans lesquelles le polymère sensible au pH se dégrade et/ou se dissout sensiblement à une valeur de pH d'environ 7,2 à environ 7,3.

11. Formes posologiques orales selon l'une quelconque des revendications précédentes, dans lesquelles le polymère sensible au pH est choisi dans le groupe constitué par des hydroxypropylméthylcelluloses et des copolymères anioniques d'acide méthacrylique et de méthacrylate de méthacryle.

12. Formes posologiques orales selon la revendication 11, dans lesquelles l'hydroxypropylméthylcellulose est l'acétate-succinate d'hydroxypropylméthylcellulose.

13. Formes posologiques orales selon la revendication 11 ou 12, dans lesquelles l'enrobage comprend une première et une seconde couche d'un polymère sensible au pH, la seconde couche enrobant la première couche.

14. Formes posologiques orales selon la revendication 13, dans lesquelles la première couche contient ou est constituée d'un copolymère anionique d'acide méthacrylique et de méthacrylate de méthyle.

15. Formes posologiques orales selon la revendication 13 ou 14, dans lesquelles la seconde couche contient ou est constituée d'hydroxypropylméthylcellulose.

16. Formes posologiques orales selon l'une quelconque des revendications 13 à 15, dans lesquelles le rapport d'épaisseur entre la première et la seconde couche est d'environ 1:10 à environ 1:50.

17. Formes posologiques orales selon l'une quelconque des revendications précédentes, dans lesquelles le délitant est choisi dans le groupe constitué d'une polyvinylpyrrolidone réticulée, d'une carboxyméthylcellulose réticulée et d'un amidon modifié.

18. Formes posologiques orales selon la revendication 17, dans lesquelles la polyvinylpyrrolidone réticulée est de la Polyplasdone.

19. Formes posologiques orales selon l'une quelconque des revendications précédentes, dans lesquelles l'entérokine est choisie dans le groupe constitué par GLP-1, PYY, GLP-2, CCK, GIP et la neurotensine, de préférence GLP-1 et PYY.

20. Formes posologiques orales selon l'une quelconque des revendications précédentes, dans lesquelles le noyau comprend une substance traceuse.

21. Formes posologiques orales selon la revendication 20, dans lesquelles la substance traceuse est la caféine.

22. Formes posologiques orales selon la revendication 21, dans lesquelles le noyau contient 60 à 70 % (p/p) de glucose et 2 à 4 % (p/p) de caféine, sur la base du poids total du noyau.

23. Formes posologiques orales selon la revendication 22, dans lesquelles le noyau contient 67 % (p/p) de glucose et 3,2 % (p/p) de caféine, sur la base du poids total du noyau.

24. Formes posologiques orales selon l'une quelconque des revendications précédentes, se présentant sous la forme d'un comprimé, d'une capsule, d'une pastille ou d'un granule.

25. Formes posologiques orales selon l'une quelconque des revendications précédentes, dans lesquelles la taille est comprise entre environ 0,6 mm et environ 1,7 mm, sur la base de la plus grande dimension de la forme posologique orale.

26. Formes posologiques orales pharmaceutiques selon l'une quelconque des revendications précédentes pour une utilisation dans la prévention et/ou le traitement d'une condition ou d'une maladie choisie dans le groupe constitué de la résistance à l'insuline, du diabète sucré de type 2, de la stéatose hépatique non alcoolique, de la stéatohépatose non alcoolique, du dysfonctionnement microvasculaire, du syndrome métabolique, de l'obésité, des maladies cardiovasculaires, de l'ostéoporose, des maladies neurodégénératives, de l'altération de la fonction gastro-intestinale et des conditions de malabsorption chez un sujet.

27. Formes posologiques orales pharmaceutiques pour une utilisation selon la revendication 26, dans lesquelles lesdites formes posologiques sont formulées de telle sorte que le au moins un composé stimulant des cellules entéroendocrines pour libérer une entérokine stimule lesdites cellules présentes dans l'intestin du sujet depuis le jéjunum terminal jusqu'à la valve iléo-caecale du sujet.

28. Procédé de préparation de formes posologiques orales pharmaceutiques selon l'une quelconque des revendications 1 à 25, comprenant les étapes consistant à :
(a) préparer un mélange comprenant au moins un composé nutritif stimulant des cellules entéroendocrines pour libérer au moins une entérokine, et au moins un délitant ;
(b) comprimer le mélange obtenu à l'étape (a) ; et
(c) appliquer au mélange comprimé au moins un enrobage comprenant au moins un polymère sensible au pH qui se dégrade et/ou se dissout sensiblement à une valeur de pH d'environ 5,5 à environ 7,5.
